# EUROPEAN PATENT SPECIFICATION

(11) **EP 3 727 630 B1**
(45) Date of publication and mention of the grant of the patent: **10.04.2024**
(21) Application number: 18833861.0
(22) Date of filing: 19.12.2018
(51) Int. Cl.: B01D 15/38, B01J 20/286, B01J 20/32, C12Q 1/6886, C12Q 1/6806

(54) **METHOD AND STATIONARY PHASE FOR ISOLATING EXTRACELLULAR VESICLES FROM BIOLOGICAL MATERIAL**
VERFAHREN UND STATIONÄRE PHASE ZUM ISOLIEREN VON EXTRAZELLULÄREN VESIKELN AUS BIOLOGISCHEM MATERIAL
PROCÉDÉ ET PHASE STATIONNAIRE POUR ISOLER DES VÉSICULES EXTRACELLULAIRES À PARTIR DE MATIÈRE BIOLOGIQUE

(30) Priority: 19.12.2017 IT 201700146281
(43) Date of publication of application: 28.10.2020
(73) Proprietor: ADVANCED EXTRACELLULAR VESICLE APPLICATIONS S.R.L., 38122 Trento (IT)
(72) Inventor: D'AGOSTINO, Vito Giuseppe, 38123 TRENTO (IT); PROVENZANI, Alessandro, 38122 TRENTO (IT); QUATTRONE, Alessandro, 38122 TRENTO (IT); ZUCAL, Chiara, 38122 TRENTO (IT); NOTARANGELO, Michela, 38122 TRENTO (IT); MODELSKA, Angelika, 38122 TRENTO (IT); PESCE, Isabella, 38122 TRENTO (IT)
(74) Representative: Valenza, Silvia
(86) International application number: PCT/EP2018/085974
(87) International publication number: WO 2019/122003

(56) References cited:
- WO-A2-2004/106361
- WO-A2-2008/045526
- CN-A- 106 622 181
- FATIN MOHD NASIR ET AL: "IMMOBILISED METAL AFFINITY CHROMATOGRAPHY (IMAC) BEADS FOR LYSOZYME SEPARATION: SYNTHESIS AND CHARACTERIZATION STUDY", MALAYSIAN JOURNAL OF ANALYTICAL SCIENCES, vol. 20, no. 3, 15 June 2016 (2016-06-15), pages 578-584, XP055500136, MY ISSN: 1394-2506, DOI: 10.17576/mjas-2016-2003-17
- JOSHUA A. BORNHORST ET AL: "Purification of proteins using polyhistidine affinity tags", METHODS IN ENZYMOLOGY, vol. 326, 1 January 2000 (2000-01-01), pages 245-254, XP055310519, US ISSN: 0076-6879, DOI: 10.1016/S0076-6879(00)26058-8

## Description

### FIELD OF THE INVENTION

The present invention relates to the field of extracellular vesicles isolation from cell culture media or biological fluids.

### BACKGROUND

Extracellular vesicles (EVs) are membranous particles that include exosomes (80-200 nm), microvesicles (100-600 µm) and apoptotic bodies (800-5000 nm). This classification is mainly based on vesicle size, although different mechanisms have been proposed for their biogenesis. In oncology, EVs hold potential to study the modulation of tumor microenvironment and immune surveillance, to capture information and/or biomarkers released from the tumor, or to be exploited as carriers of therapeutics.

Biological and biomedical research is increasingly focused on the role of EVs in different physiological and pathological processes. Therefore, many techniques for EV isolation from biological material have been proposed to date, although many of them are not very efficient or standardizable (Théry C, et al., Curr Protoc Cell Biol, 2006; Gardiner C, et al. Extracell Vesicles 2016).

The techniques so far reported for EV isolation largely relate to the exosome purification, i.e. smaller EVs with a size between 50 and 200 nm,. The techniques widely cited are reported as follows:
- differential ultracentrifugation (Livshits et al., Sci Rep. 2015);
- density gradient centrifugation (Van Deun et al., J Extracell Vesicles, 2014; Iwai et al., J Extracell Vesicles., 2016);
- microfiltration (Merchant et al., Proteomics Clin Appl. 2010; Grant et al., J Immunol Methods, 2011; Hyun-Kyung Woo et al., ACS Nano. 2017);
- microfluidics (Davies et al., Lab Chip, 2012; He et al., Lab Chip, 2014; Kanwar et al., Lab Chip, 2014; Liga et al., Lab Chip, 2015);
- immunoprecipitation using synthetic peptides (Ghosh et al., PLoS One, 2014), heparin (Balaj et al., Sci Rep. 2015), combination of antibodies that recognize membrane (Pugholm et al., Biomed Res Int. 2015; Cao et al., Mol Cell Proteomics, 2008) proteins (cluster differentiation or antigens or components), or Tim4 protein (Nakai et al., Sci Rep. 2016);
- polymer- or solvent-based precipitation/isolation (Deregibus et al., Int J Mol Med. 2016; Taylor et al., Methods Mol Biol., 2011; Gallart-Palau et al., Sci Rep. 2015);
- ultrasounds (Lee et al., ACS Nano 2015);
- commercial systems based on columns including ExoQuick^{™} (System Bioscience), Total exosome isolation reagent (Thermo Fisher), miRCURY^{™} exosome Isolation kit (Exiqon), exoEasy (Qiagen), Exo-spin^{™} (Cell Guidance), ME^{™} kit (NEP).

Ultracentrifugation is now considered the most effective and widely applied (gold-standard) procedure as a primary isolation method (Gardiner C et al, J Extracell Vesicles, 2016; Al-Nedawi K and Read J Methods Mol Biol. 2016), due to the fact that alternative density gradient centrifugations and precipitation techniques use chemical agents interfering with EV yield, composition and integrity; immunoaffinity capture leads to differential isolation of EV subpopulations (hence low heterogeneity) and it is expensive because it involves antibodies; exclusion chromatography requires significant volumes of biological sample giving a very low yield; column- and centrifuge-based systems strongly damage EV integrity. However, there are critical issues even in ultracentrifugation: they concern laboriousness, important sample volume to be processed and timing (6-12 hours), purity of the resulting obtained sample, instrument used, operator experience, high level of contaminants (protein aggregates) co-sedimenting with EVs, degradation of biomolecules due to the processing time (Lobb et al, J Extracell Vesicles 2015, Gardiner C et al, J Extracell Vesicles, 2016).

At the state of the art, Ni²⁺-functionalized stationary phases are known (such as Immobilized Metal Ion Affinity Chromatography (IMAC), nickel chelate acceptor beads, Dynabeads) designed for the purification or recognition of recombinant proteins with histidine tag. In these functionalized stationary phases, the positive net charge deriving from functionalization is hardly ever described by manufacturer, reporting instead the stability index at different pH (very variable), which influences binding efficiency in solution to proteins with a broad range of size (from a few to hundreds kDa).

The purpose of the present invention is to provide a suitable functionalized stationary phase, and a related method of functionalizing and exploiting it, as a new instrument for isolating extracellular vesicles (EVs); said method must be faster, less laborious and more efficient than the ultracentrifuge, and presents further advantages compared to current methods.

### SUMMARY OF THE INVENTION

The present invention provides a stationary phase which can be consisting of magnetic or non-magnetic particles, of micrometric or nanometric size, functionalized with Ni²⁺ or Al³⁺ cations and characterized in that it has a positive net charge between 30 and 80 mV, associated with efficiency on heterogeneous EV isolation as shown below.

The stationary phase (e.g. agarose beads) according to the invention allows a rapid and efficient isolation of whole EVs characterized by a wide range of dispersity with a size range between 50 and 2000 nm that therefore allows to capture both exosomes and microvesicles in the biofluids.

In an aspect, the present invention relates to a method for preparing the stationary phase as described above, said method comprising suspending a non-functionalized stationary phase in a saline solution buffered at a physiological pH containing from a minimum of 15 mM to a maximum of 100 mM (based on the stationary phase capacity and in order to obtain the efficiency shown below on the isolation of heterogeneous EVs) of a Ni²⁺ or Al³⁺ salt, and in any case according to the capacity of the stationary phase employed and the net positive charge obtained. In an aspect, the present invention relates to a method for isolating EVs secreted by eukaryotic or prokaryotic cells (bacteria) in culture media or biological fluids, said method comprising the use of the stationary phase functionalized with nickel ions as described above. The method of the invention is hereinafter referred to as NBI (nickel-based isolation).

Extracellular vesicles (EVs) present physicochemical properties, such as structure, size, buoyant density, optical properties and electrokinetic potential (zeta potential) depending on their lipidic double layer structure and lipid and protein composition (Yáñez-Mó M et al, J Extracell Vesicles 2015).

The principle of the method of the invention presented here is based on the exploitation of EV electrokinetic potential (hereinafter referred to as ZP) in combination with a stationary phase (agarose, silicon, magnetic beads, etc.) functionalization with nickel ions for their isolation and purification.

Several reports recently published show that in physiological solution of PBS the ZP of extracellular vesicles is between -17 and -35 mV (Rupert DL et al, Biochim Biophys Acta 2017), an index of moderate-good stability and good dispersity (Correia et al., Langmuir, 2004).

The advantages of the NBI method, according to the invention, with respect to the gold standard technique (UC) used for EV purification are:
- rapidity: application time of less than 60 minutes;
- simplicity: the use of specific instruments is not necessary with a few procedural steps;
- adaptability: the quantity of functionalized beads is adapted to the volume of the starting biological material (from tenths of a milliliter to liters);
- efficiency: high recovery of intact EVs;
- heterogeneity/dispersity: simultaneous precipitation of heterogeneous EVs (dimensional range typically between 50 and 800 nm) with limited aggregation phenomena;
- stability: the polydispersed EVs purified by NBI are more stable than those purified by UC;
- label/polymer-free: absence of hydrophobic polymers added to the starting biological sample or during the whole procedure of NBI;
- physiological pH: the whole procedure is carried out with salt solutions buffered at pH 7.4;
- purity: NBI allows to selectively purify EVs from biological fluids enriched in proteins;
- combination: NBI can be coupled to other systems designed to isolate EVs and based on other physicochemical principles, especially in order to obtain EV subpopulations with different size coming from the same starting biological sample;
- versatility: NBI is applicable to biological samples exposed to protein- or nucleic acid-degrading enzymes, such as trypsin, proteinase K, DNAses, different RNAses; possibility of labelling the biological sample with fluorescent lipophilic probes after processing with NBI.
- tolerability for *in vivo* injection/ infusion of extracellular vesicles purified with NBI. In an aspect, the present invention also relates to a kit comprising:
   a container containing a stationary phase as described above.

### DETAILED DESCRIPTION OF THE INVENTION

The stationary phase according to the invention is preferably functionalized with nickel.

The stationary phase is preferably selected from the group consisting of agarose or silicon beads, whether magnetic or non-magnetic, alginate salt matrices, polymers for Immobilized Metal ion Affinity Chromatography (IMAC), nickel chelate acceptor beads, anionic (as styrene divinylbenzene) or carbon (as graphene) styrene polymers. Agarose beads are particularly preferred.

By micrometric size we mean from 0.5 to 1000 µm; by nanometric size we mean from 1 to 500 nm.

The method of the present invention is hereinafter described on the basis of an embodiment with agarose beads of known nominal size (preferably 25-40 µm) functionalized with nickel ions, i.e. positively charged, therefore allowing the binding in a physiological solution to negatively charged nanoparticles and microparticles. The quantity of nickel ions exposed to the concentration of beads used confer to them electrochemical properties resulting in a positive net charge between 30 and 80 mV, stable for at least six months if the beads are stored at 4°C, in physiological phosphate buffer saline (PBS) solution. Any preservatives such as sodium azide or 20% ethanol can be added to the storage solution, after extensive washing in PBS prior to use the stationary phase.

The saline solution at physiological pH is preferably PBS, but may be substituted with any other buffered solution (Trizma Base, HEPES, etc.) not interfering with divalent cations, e.g. nickel, (in transition or stabilized in detectable forms) and buffered at physiological pH 7.4. The buffered saline solution containing Ni²⁺ ions is preferably further sterilized with 0.2 µm filters.

The agarose beads according to the invention are preferably prepared from non-functionalized beads by incubation in a 15-30 mM solution of a nickel salt (preferably nickel sulphate, or nickel chloride, nickel oxide, or, in the case of functionalization with aluminium, aluminium sulphate, aluminium chloride, aluminium oxides and/or aluminium silicates) in PBS at pH 7.4, sterilized with 0.2 µm filters.

Preferably the mixture of beads in the nickel salt solution is incubated at room temperature (20-25° C) and in gentle orbital rotation for a minimum of 1-3 minutes. After incubation the beads are separated from the solution by centrifugation.

The supernatant is removed and the beads washed with a buffered saline solution, preferably sterilized. Preferably, washing may be repeated 2-3 times to remove nickel ion traces and residual counter-ions by suspension, centrifugation and supernatant removal.

After washing the beads are preferably suspended in a volume, preferably equal to that of the beads, of saline solution buffered at physiological pH, preferably sterilized with 0.2 µm filters, and they (hereinafter referred to as CBeads) can be stored at 4°C.

If the stationary phase is (as commercially available) functionalized with Ni²⁺ or Al³⁺ cations, before functionalization as described above, it is necessary to subject it to stripping by one or more washing with an aqueous solution supplemented with 200-300 mM NaCl or KCI, 100-300 mM EDTA or EGTA, 300-500 mM Imidazole, or a solution containing cationic chelating agents with a wide pH range (generally between 5 and 8), and one or more washing with bi-distilled water (18.2 MΩ cm⁻¹). The method according to the present invention involves the use of CBeads which can be added dropwise to the surface of a biological liquid preferably clarified by cellular debris by centrifugation at 2800 rcf, collected in tubes of any size and incubated at room temperature for a minimum 30 minutes. CBeads are added in a volumetric ratio of 10-30 µl for each ml of sample, and an excess thereof is empirically established on the basis of the number of particles found in the biological fluid.

The biological liquid can be:
- cell culture medium containing a max of 1.5% of fetal bovine serum (FBS) to be used as such for the NBI procedure. If the percentage of FBS is higher, it is preferable to dilute with PBS, at pH 7.4;
- bacterial culture media (LB, or containing peptones, sugars and/or yeast extracts);
- physiological buffer solution;
- liquid biopsy sample (whole blood or serum or plasma, urine, cerebrospinal fluid, milk, saliva, tissue exudates) from human or animal origin. Even in this case the viscosity of the medium can be diluted with a suitable PBS volume.

After incubation with the biological sample CBeads+EVs are separated by decantation. A weak centrifugation at a maximum speed of 300 rcf is allowed to speed up the step and, at the same time, to preserve EVs and stationary phase integrity during the NBI procedure.

The detachment of EVs from the CBeads is carried out by adding a solution (from now on defined Elution), freshly prepared in PBS at pH 7.4 before use, obtained by mixing two solutions A and B containing at least 2 different chelating agents.

Said solution A preferably contains 3-6 mM EDTA at pH 8.0; more preferably solution A is PBS supplemented with 3-6 mM EDTA at pH 8.0.

Said solution B preferably contains 30-300 µM sodium citrate; more preferably the solution B is PBS supplemented with 30-300 µM sodium citrate and 50-100 mM NaCl. For both solution A and B, other chelating agents that can be used are Imidazole, DTPA, NTA, amidoxime, molecules designed for the purpose or peptides which can establish covalent and/or competitive binding with Ni²⁺ and, therefore, promoting a step of EV release from the stationary phase more or less efficient. Once the solutions A and B have been mixed, a suitable amount of KH₂PO₄ is added (8µl of KH₂PO₄ are added when EDTA is 3.2 mM, NaCl is 60 mM and sodium citrate is 45 µM) or of any other saline solution buffering to the physiological pH 7.4 and at the same time, not interfering with nickel ions and/or with the morphological-biochemical properties of the EVs.

The CBeads+EVs are preferably incubated with at least the same volume of Elution solution, said incubation preferably in orbital rotation for a minimum of 10-20 minutes at 20-37°C, preferably 28°C.

To separate the CBeads from the Elution+EV solution it is preferable to centrifuge in a tilting rotor at a minimum of 300 rcf for about 1 minute. The supernatant containing whole and polydispersed EVs (generally distributed between 50 and 2000 nm), is transferred into preferably low-binding sample tubes.

The above description with reference to agarose beads is valid, with obvious adjustments, for any other stationary phase according to the present invention. The aforesaid procedure, described in all its steps, may be supported by further modifications or instrumental couplings suitable to contain the stationary phase, of whatever nature according to the biochemical conditions specified above, in conditions of stability or mobility (columns, matrices, filters, etc.) and which allows for a simplification or procedural optimization.

The method described above is compatible with downstream applications such as RNA extraction from EVs, EV precipitation and/or sorting based on antibodies, amplification of nucleic acids contained or adsorbed in EV by PCR (polymerase chain reaction) and technical variants thereof, transfection of EV in eukaryotic cells, EV engineering with nucleic acids, EV exploitation as nucleic acid, peptide or pharmacological agent carriers.

In an aspect, the present invention relates to a kit comprising:
a container containing a stationary phase as described above or, alternatively,
a container containing a non-functionalized stationary phase consisting of magnetic and non-magnetic particles of micrometric or nanometric size; and
a container containing a Ni²⁺ or Al³⁺ salt.

Preferably the kit further comprises:
a container containing a saline solution buffered at physiological pH;
at least two containers each containing a different chelating agent.

Preferably, the kit further comprises:
at least one container containing an adjusting pH saline solution buffering at physiological pH the mixture of chelating agents.

Preferably, the kit can further comprise:
microcentrifuge low-binding sample tubes.

The kit described above wherein preferably:
the stationary phase consists of 25-40 µm agarose beads;
the nickel salt is NiSO₄ or the aluminium salt is Al₂O₃ or aluminosilicates;
the saline solution buffered at physiological pH is PBS;
a chelating agent is EDTA when the cation is Ni²⁺, or magnesium salts such as MgCl₂ or MgSO₄ when the cation is Al³⁺;
the other chelating agent is sodium citrate when the cation is Ni²⁺, or calcium salts such as CaCl₂ or Ca₃(PO₄)₂ when the cation is Al³⁺;
the pH adjusting saline solution is KH₂PO₄ or K₂HPO₄.

The present invention will be better understood in light of the following embodiments.

### BRIEF DESCRIPTION OF THE FIGURES

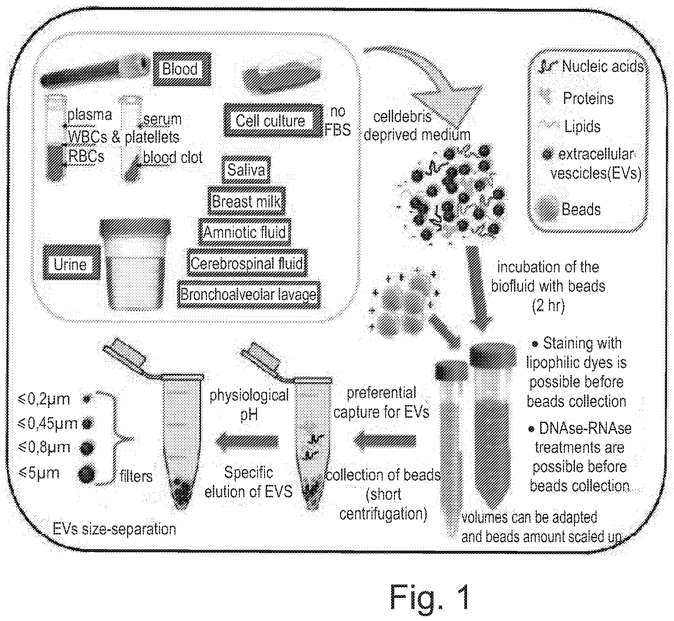

FIG. 1- Scheme indicating the user-friendly approach and the steps for EV isolation by NBI according to the present invention starting from biological material of different origin.

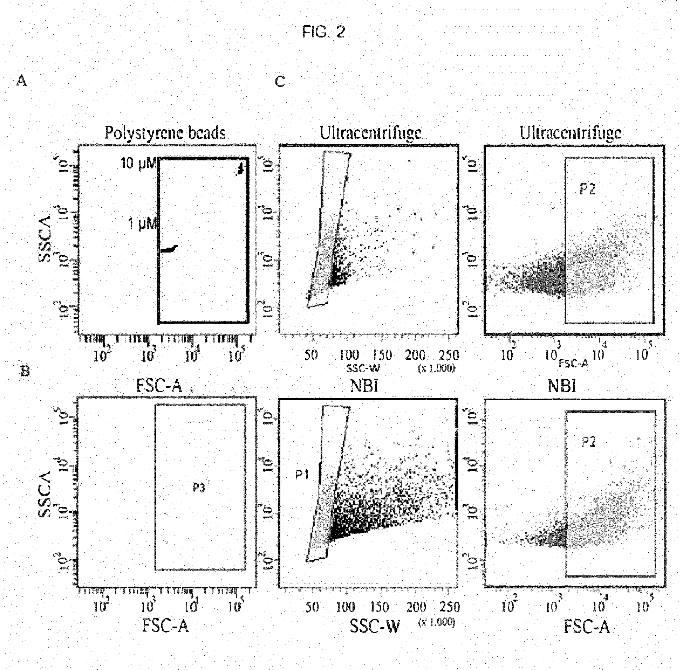

FIG. 2 - Flow-cytometry analysis showing the comparison between the number of particles (> 0.5 µm) isolated by ultracentrifugation or by NBI. **A**) The FACS Canto (BD) instrument was calibrated with 1 and 10 µm diameter polystyrene beads (F13838, Life Technologies). **B**) Number of particles captured using NBI procedure using non-functionalized beads (negative control). **C**) Number of particles >0.5 µm (P2) obtained after ultracentrifuge (56.6%) or NBI (58.8%) using agarose beads functionalized with 19 mM nickel sulphate. Graphs are representative of two independent experiments.

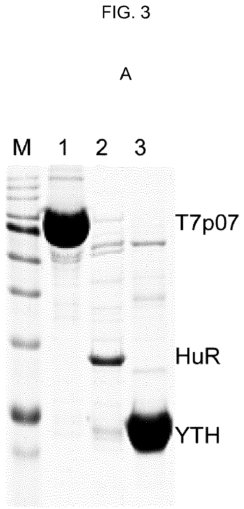

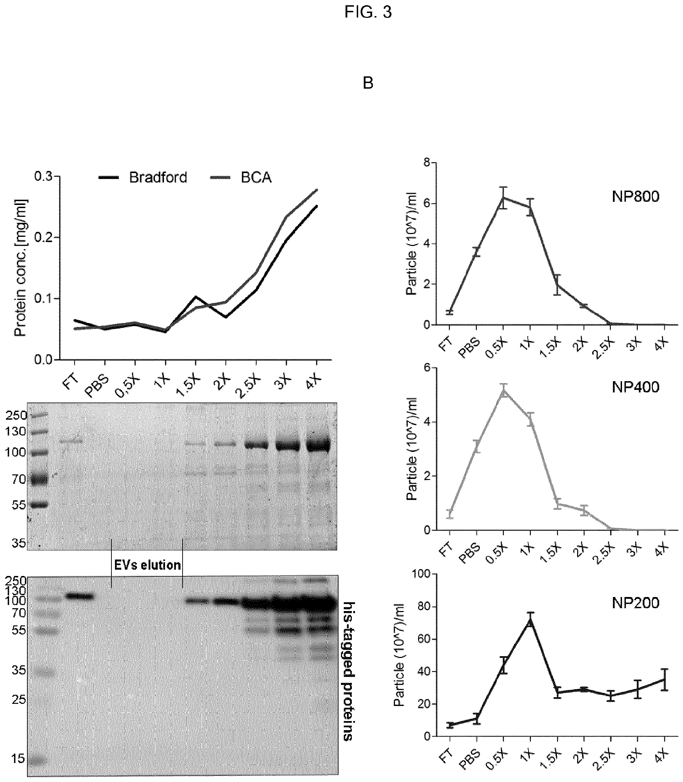

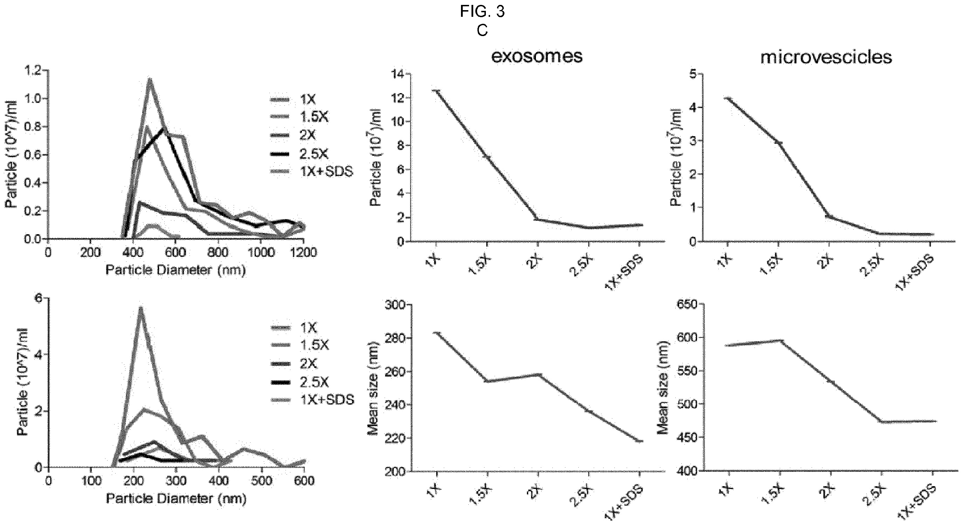

FIG. 3 - **A**) Purification of proteins with histidine tag. Coomassie staining after 15% -SDS polyacrylamide gel of recombinant proteins shows: M: pre-coloured protein ladder; 1: T7p07 protein, 105 kDa (2 µg loaded on gel); 2: HuR protein, 36 kDa (300 ng loaded on gel); 3: YTHDF1 protein, 23 kDa (2 µg loaded on gel). **B**) Competitive assay performed in serum-free cell culture medium enriched with 500 µg/ml of E. coli DH5α total protein extract and with a total of 150 µg/ml of recombinant proteins shown in **A** (T7p07, HuR, YTHDF1). Two parallel gels were developed after SYPRO Ruby staining or western blotting using an antibody against the histidine tag. FT (flowthrough) is the medium after exposure to NBI beads and the graphs result from the analysis with qNANO (iZON Science) using the indicated nanopores. The 1X solution allows for maximum selective elution of EV in a protein enriched system. **C**) Increased concentration of chelating agents and salts in the elution buffer alters EV integrity over 1.5X. The graphs obtained by qNANO analysis are representative of two independent experiments and the decrease in number and size indicates EV detrimental effect.

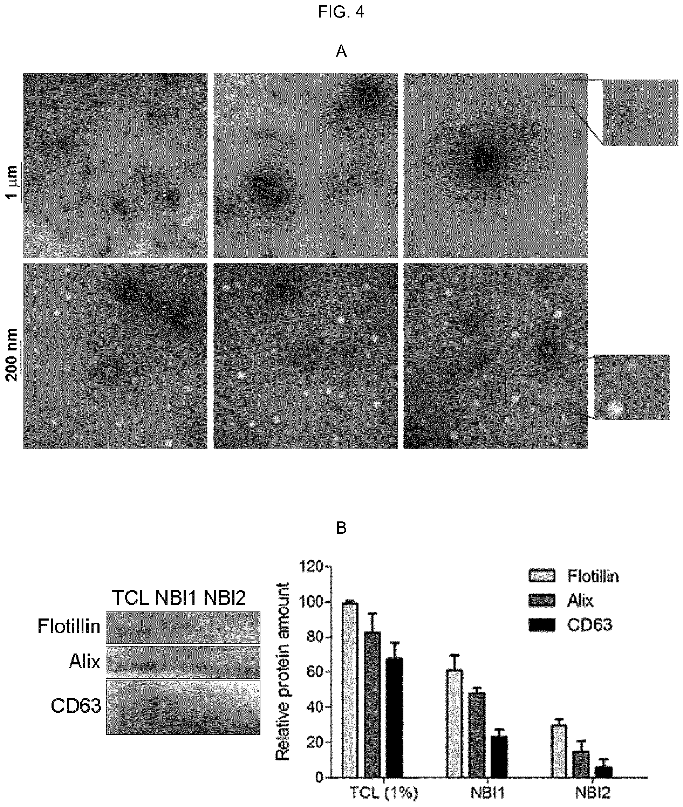

FIG. 4 - **A**) Transmission electron microscope (TEM) acquisitions of EVs obtained by NBI after fixation with 2.5% paraformaldehyde in elution buffer. **B**) Western blotting analysis of recovered EV lysates from 18*10⁶ (NBI1) or 9*10⁶ (NBI2) or 1% of U87 cell total cell lysate (TCL).

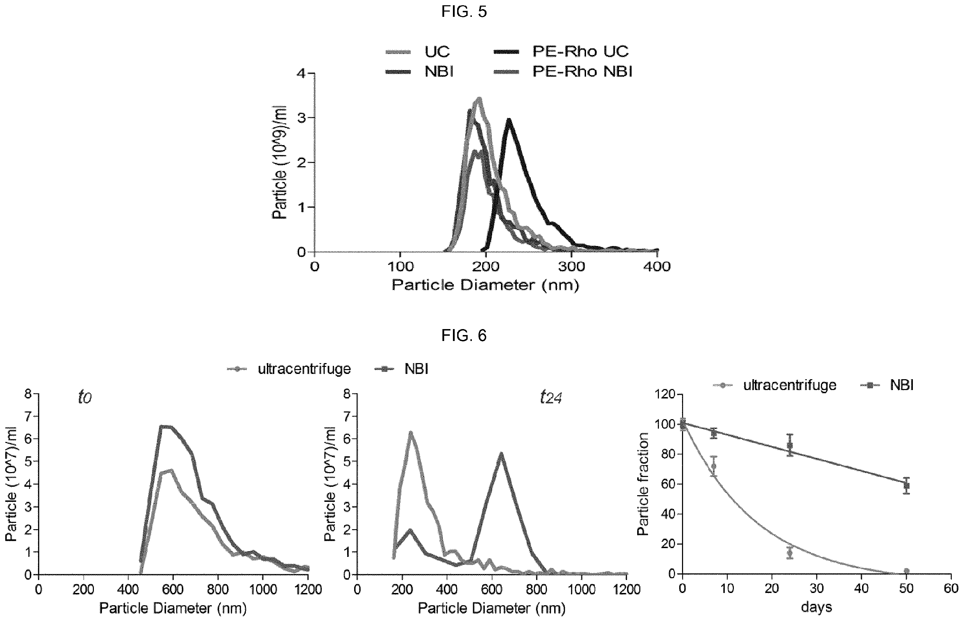

FIG. 5 - Mixture of 4.2*10⁹ liposomes with 181±23.8 nm diameter recovered by ultracentrifugation (UC) or NBI. The same experiment was replicated after liposome staining with phosphatidylethanolamine-rhodamine (PE-Rho, 5 ng/ml).
FIG. 6 - Microvesicles isolated from U87 cells cultured in 3 ml of serum-free medium and analysed immediately after (t0) or after 24 days (t24, stored at 4 ° C) by UC or NBI. Repeated gNANO measurements were then performed up to 52 days post-purification and the half-life of the microvesicle population was calculated using GraphPad Prism software v.5 compared to time *t0.*

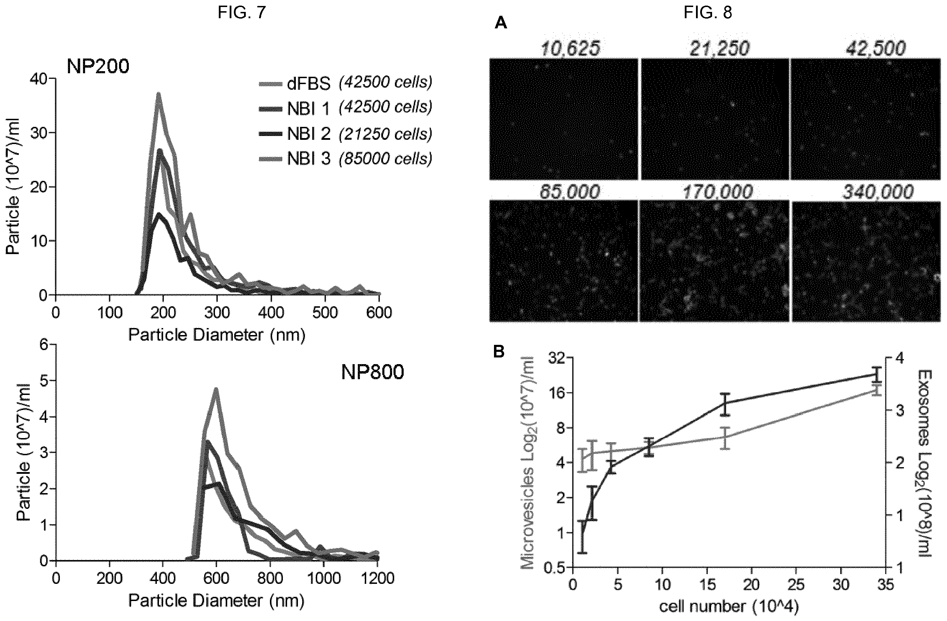

FIG. 7 - The reproducibility of NBI method was tested using serum-free medium by U87 cells seeded at different density, in 6-well plates, as indicated (NBI 1, 2, and 3). The 10% ultracentrifugate serum condition (dFBS) showed substantial overlap with the results obtained with the NBI1 samples. Graphs are representative of 3 independent experiments and the overall coefficient of variation (CV) was 6.1%, demonstrating the excellent reproducibility of the method.
FIG. 8 - Isolated EVs as a function of cell density in 6-well plates (1 ml) reveal different release dynamics in the culture medium. **A**) EVs were purified by NBI after 24 hours and the cells were exposed to 10 ng/ml Hoechst33342, a DNA stain indicating the number of seeded cells. The images were acquired with a "high content" analysis system, Harmony software v4.1, and the Operetta instrument (Perkin Elmer). **B**) Media described in A were processed by NBI and the number of exosomes and microvesicles, here indicated in Log2, was measured with qNANO (iZON Science). The standard deviation is here referred to 3 independent experiments.

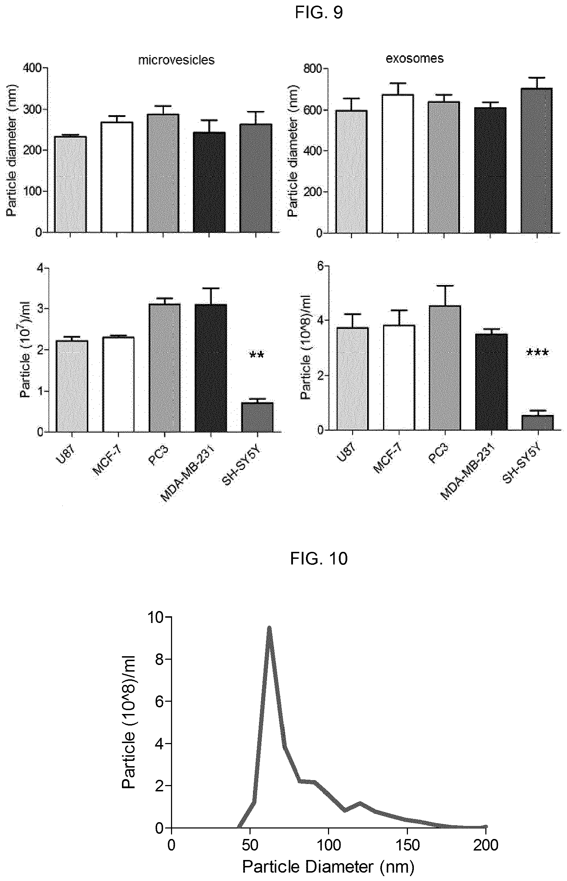

FIG. 9 - Number and size of EVs isolated by NBI were analysed for several tumor cell lines, as indicated. Only SH-SY5Y cells showed substantial reduction in the release of both EV populations (**P value=0.006, F=70.13, and ***<0.0001, F=30.84, using ANOVA and Bonferroni post-test). The standard deviation is here referred to 3 independent experiments.
FIG. 10 - NBI versatility has also been confirmed in isolating vesicles released by Gram-negative bacteria. E. coli DH5α cells were grown in whole LB medium up to OD₆₀₀ = 0.7. Bacteria were pelleted by centrifugation at 4000 rcf for 15 min and the supernatant was used for NBI processing. The particles were analysed with qNANO using NP150 and NP200 nanopores and the largest detected population showed 92±29 nm in diameter. Graph is representative of two independent experiments.

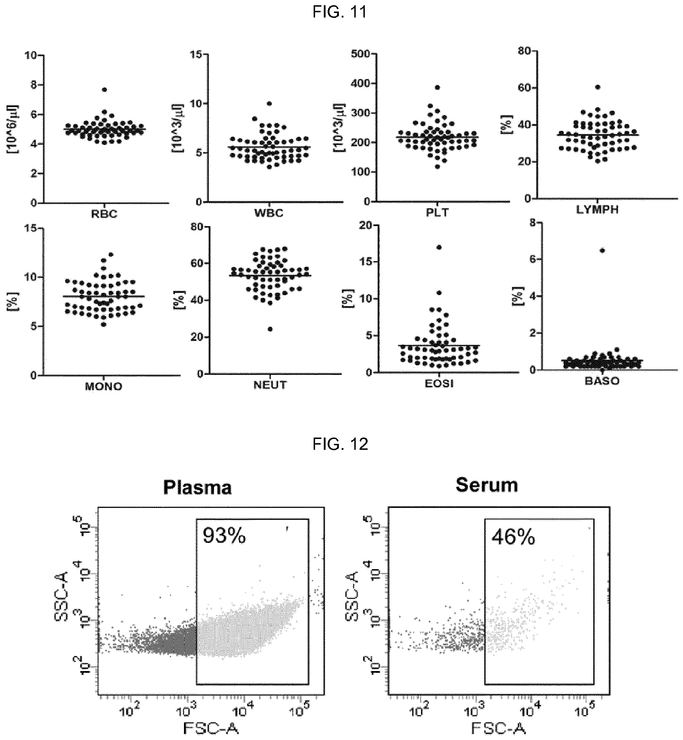

FIG. 11 - Blood corpuscular element counting, taken from 47 healthy donors with an average age of 45 years at the Meyer Children's Hospital of Florence, was carried out by Sysmex XE-5000 hematology analyser (Sysmex America, Mundelein, IL) according to manufacturing instructions.
FIG. 12- Comparison between number of microparticles (≥500 nm) isolated by NBI from same donor plasma and serum. Flow cytometer events were acquired for 2 minutes and the relative percentage of the same particle population is shown.

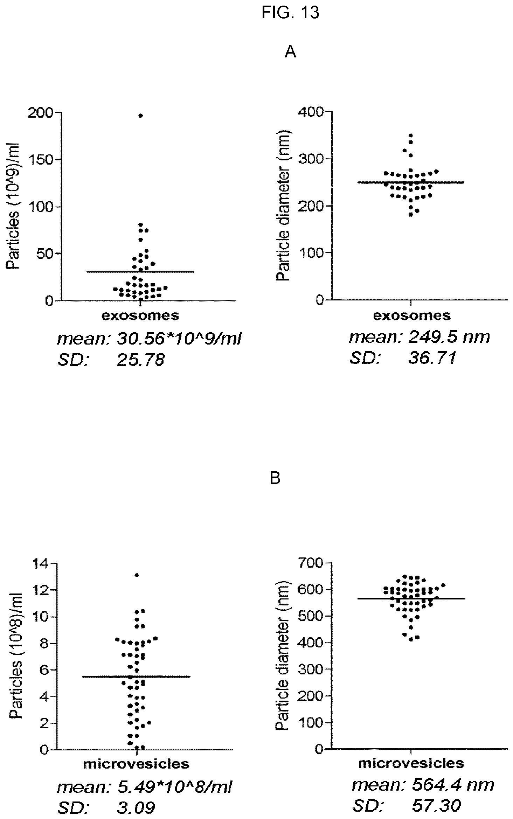

FIG. 13 - **A**) NBI was applied to 0.5 ml of plasma and TRPS analysis detected number and size of indicated vesicles. The number of exosomes was correlated with erythrocytes (RBC), Pearson coefficient = 0.99. **B**) The same analyses carried out in A were applied for the microvesicle count and the correlation with platelets (PLT) was 0.98.

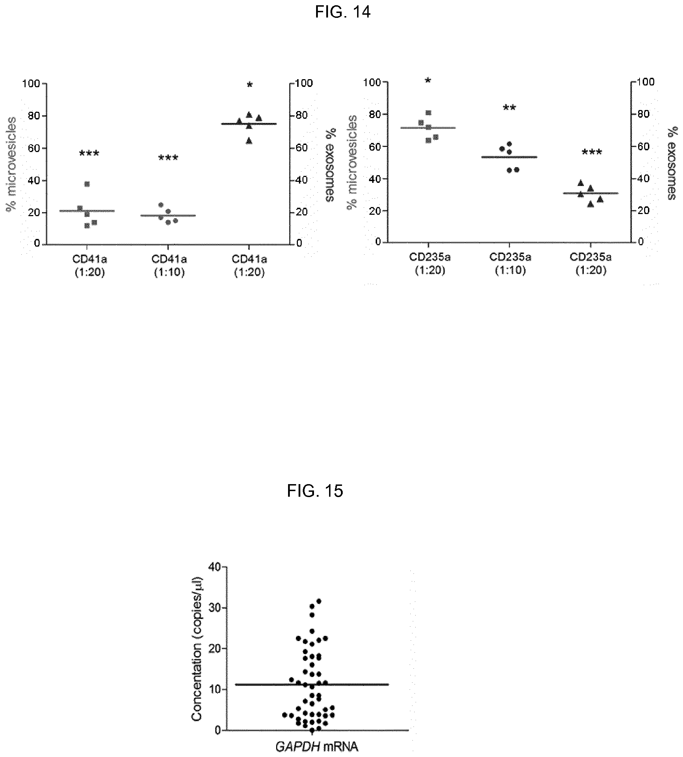

FIG. 14 - CD41a and CD235a biotinylated antibodies, directed against known platelet or globule markers, respectively, were exploited to explore "EV lineages". Each sample of vesicles in solution purified by NBI was divided into two equivalent parts to be respectively incubated with antibody or biotin (negative control). After precipitation with streptavidin-conjugated magnetic beads, the remaining vesicles in solution were characterized at qNANO. The number of particles shown is normalized to the relative negative control. **P value* <0.05; ***P value* <0.01; ****P value* <0.001.
FIG. 15 - Two microliters out of 20 of cDNA synthesized starting from 0.1 - 4 ng RNA extracted from NBI-purified vesicles from the plasma of 47 donors. Droplet digital PCR (ddPCR) was used with EvaGreen chemistry and the following primers: 5'-CAACGAATTTGGCTACAGCA (SEQ ID No. 1) and 5'-AGGGGTCTACATGGCAACTG (SEQ ID No. 2). The absolute number of copies of GAPDH mRNA was obtained after analysis with QuantaSoft Analysis software (BIORAD).

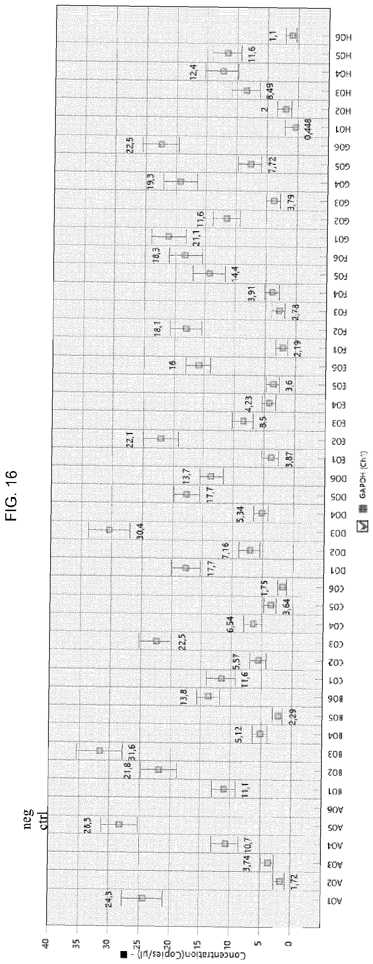

FIG. 16 - FIG. 17- The absolute number of copies of GAPDH mRNA reported in Fig. 15 is positively correlated with the number of platelets (r = 0.62).

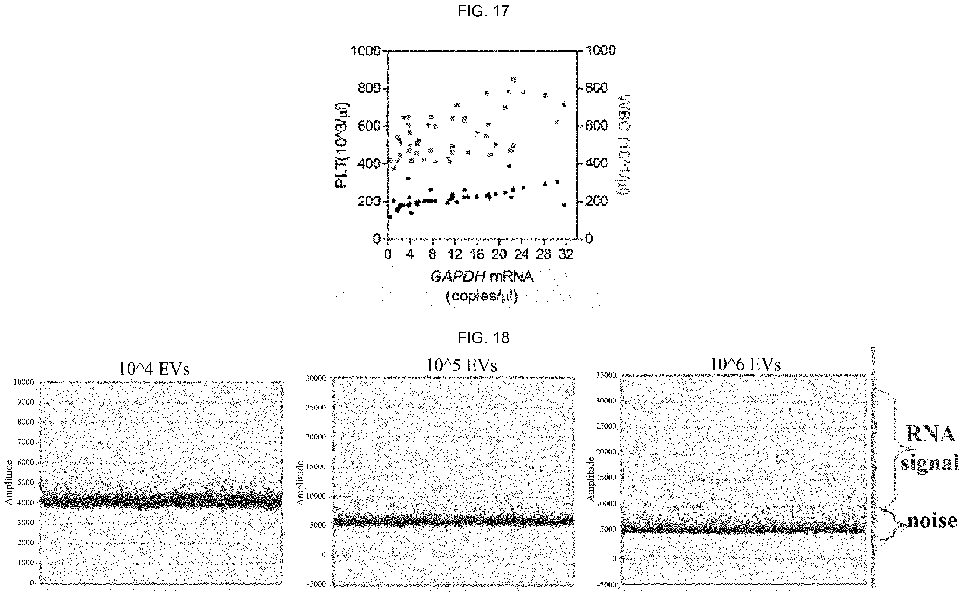

FIG. 18 - Buffer solutions used in the NBI method are compatible with droplet digital PCR (ddPCR), directly used on whole EVs purified from SK-MEL-28 melanoma cells for quantitative analysis of specific RNA containing the V600E mutation .

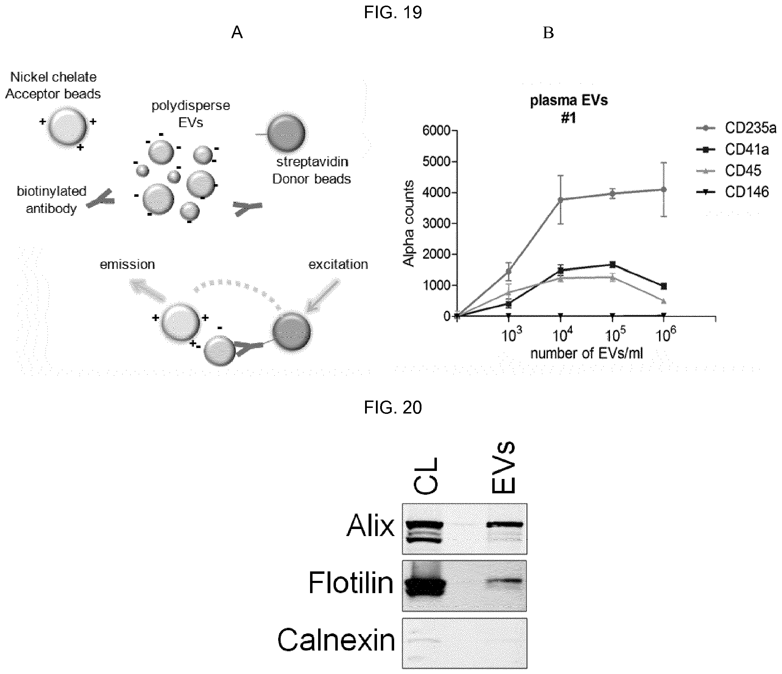

FIG. 19 - A) initial binding between charges of opposite sign between nickel (positive) and EV (negative) makes possible the coupling between NBI and Alpha technology, where nickel-Acceptor beads are used in combination with biotinylated antibodies and streptavidin beads -Donor for capture and detection of specific antigens on the surface of EV. B) Example of recognition and quantification of specific antigens (CD235a, CD41a, CD45) present on EV circulating in human plasma. The CD146 antigen is a marker for cells of epithelial origin that acts as an experimental negative control.
FIG. 20 - The buffer solutions used for NBI are also compatible with conventional techniques for detecting proteins in denaturing conditions, such as western blotting.

### EXPERIMENTAL SECTION

### EXAMPLE 1- Preparation of the nickel ion functionalized beads

The procedure for beads functionalization is carried out as follows:
- commercially available NiNTA Sepharose High Performance beads (GE Healthcare product code 71-5027-67 or 17-5268-01) was taken as starting beads; the net charge values were measured and resulted in the range from 3 to 25 mV at 22-25°C in PBS, as detected using the Malvern Zetasizer instrument.
- starting beads are subjected to stripping by one or more washing with an aqueous solution supplemented with 200-300 mM NaCl or KCI, 100-300 mM EDTA or EGTA, 300-500 mM Imidazole, or a solution containing cationic chelating agents with a wide pH range (generally between 5 and 8), and one or more washing with bi-distilled water (18.2 MΩ cm⁻¹);
- A 40 mg/ml suspension of starting beads (NiNTA Sepharose High Performance Beads, GE Healthcare, 17-5268-01, 34 µm known nominal size), previously subjected to stripping treatment aliquoted in 50 ml tubes is re-suspended in a double volume (related to the bead volume) of a concentrated 19 mM nickel sulphate solution in PBS at pH 7.4, sterilized with 0.2 µm syringe filters.

- The bead mixture in the nickel sulphate solution is incubated at room temperature and with gentle orbital rotation for two minutes.
- The 50 ml tube is centrifuged in a tilting rotor at 200 rcf for 1 minute and the beads are collected at the bottom of the tube.
- The supernatant is gently sucked up and discarded and a triple volume (related to the bead volume) of PBS at pH 7.4 sterilized with 0.2 µm syringe filters is added to the beads.
- The beads are centrifuged again as described, the supernatant is sucked up and discarded.
- The step of PBS addition and removal to the beads is sequentially repeated two more times to remove residual sulphate or nickel ion traces.
- The beads are re-suspended in the same volume (related to the bead volume) of PBS at pH 7.4, sterilized with 0.2 µm syringe filters, and these beads (hereinafter referred to as CBeads) stored at 4°C.

The quantity of nickel ions exposed to the beads gives them electrochemical properties resulting in a positive net charge between 40 and 60 mV, stable for at least six months at room temperature, in phosphate buffer saline (PBS) physiological solution.

### EXAMPLE 2- EV isolation method from a biological sample

CBeads can be added dropwise to the surface of a biological liquid (clarified by cellular debris by 2800 rcf centrifugation) collected in tubes of any size and incubated at room temperature for 30 minutes in a volumetric ratio of 20 µl/ml.

Biological fluid may be cell culture medium mostly containing 1.5% fetal bovine serum (FBS)-PBS at pH 7.4 dilution is allowed if FBS percentage is higher; liquid biopsy sample (whole blood or serum or plasma, urine, cerebrospinal fluid, milk, saliva).

EV isolation from the biological sample is carried out as follows:
- CBeads are incubated with the biological sample with gentle orbital rotation (300-600 rpm) for 30 minutes at room temperature, at the end of which the tube is stabilized in a vertical position to allow gravity settling or weak centrifugation (100-400 rcf) of the CBeads (7-15 minutes) at the bottom of the tube.
- The supernatant is completely sucked up and discarded.

EV purification, i.e. their removal from the beads, is promoted by a solution (defined from now on Elution) prepared a few minutes before use in PBS at pH 7.4, given by mixture of two solutions A and B containing chelating agents.

EV-Elution A: PBS supplement with final of 3.2 mM EDTA pH 8.0.

EV-Elution B: complete PBS with 60 mM NaCl, 45 µM sodium citrate.

Once the EV-Elution A and B buffers are mixed (Elution 1X solution is obtained), 8 µl/ml KH₂PO₄ are added to the Elution solution just before the EV elution.

Elution solution allows an ion exchange among the elements in solution and promotes a rapid EV separation from the agarose beads, while preserving EV integrity, size and morphology.

EV purification is carried out as follows:
- A volume equal to that of the CBeads of Elution solution is dropwise and gently added to the CBeads.
- The tube containing CBeads+Elution mixture is incubated with orbital rotation (500 rpm) for 15 minutes, preferably at 28°C.
- The tube is centrifuged in a tilting rotor at 1800 rpm for 1 minute.

The supernatant, containing whole and polydispersed EVs (generally distributed between 50 and 800 nm), is transferred to low-binding tubes.

### EXAMPLE 3 - Comparison of particle number (> 0.5 µm) isolated by UC or by NBI

NBI was applied to isolate vesicles released from U87 gliomas cells and the particle number with ≥0.5 µm in diameter (as estimated by flow cytometry) is comparable to that obtained using differential UC, unlike few events captured by non-functionalized beads (Fig. 2A).

### EXAMPLE 4 - Analysis of particle populations and refinement of the elution step

In order to analyse particle populations and refine the elution step that allowed EV enrichment in solution, the tunable resistive pulse sensing (TRPS) was systematically used with the qNANO instrument.

In order to evaluate selectivity of elution, competitive tests were performed in a protein enriched system, including recombinant proteins with 6X histidine, a tag known to confer the strongest interaction with Ni²⁺. The serum-free medium of U87 cells, complemented with raw extracts of DH5α E. coli cells (500 µg/ml) and with different purified proteins (50 µg/ml each, T7p07, 105 kDa, HuR, 36 kDa; YTH, 23 kDa, Fig. 3A), was subjected to NBI following a buffer elution gradient, keeping Elution 1X solution (3.2mM EDTA, 45 µM sodium citrate and 60mM NaCl) as reference. The highest molecular weight (MW) proteins, enriched in T7p07 with minimal amounts of HuR and YTHDF1, were progressively eluted, starting with a 1.5X Elution solution (4.8 mM EDTA, 90 mM NaCl and 67.5 µM) NaCitr), as demonstrated by SDS-PAGE, Ruby SYPRO staining and western blotting using anti-His antibody (Fig. 3B). In contrast, the majority of EVs were eluted with 1X elution buffer, demonstrating that a reduced amount of chemical agents is sufficient to displace EV/Ni-bead interactions without nickel contamination in solution, an event requiring> 100 mM EDTA. In particular, EV morphology changed according to the different elution buffers, which influenced their size and dispersion starting from the 1.5X solution (Fig. 3C).

Transmission electron microscopy (TEM) (20500x and 87000x magnifications, Fig. 4A) confirmed a low protein level in the NBI samples and indicated that 1X solution retained EV morphology and wide dispersion, showing 541 ± 120 nm in diameter and a dispersity index of 0.61 ± 0.05 as assessed by the dynamic light scatter (n = 3). These data were coupled with western blotting analysis for positivity to membrane-associated or endosomal proteins in EV lysates resulting from a small number of 9*10⁶ U87 cells (Figure 4B), confirming NBI effectiveness in heterogeneous EV recovery.

### EXAMPLE 5 - Impact of mechanical forces and salts equilibrium during the NBI procedure

To analyse the impact of mechanical forces and salts equilibrium during the NBI procedure, liposomes similar to exosomes were produced at four different mean sizes (149, 177, 196, 202 nm) and a mixture thereof was added in 10 ml of DMEM medium before processing with NBI (Figure 5). Both NBI and UC allowed complete recovery (98.6%) of particles. In contrast, we observed that liposomes pre-stained with phosphatidylethanolamine-rhodamine and ultracentrifugated (PE-Rho UC) had coalescence behavior (> 40 nm displacement) in contrast to PE-Rho NBI particles, probably due to a greater membrane damage or curvature generated by UC. These data indicate that NBI better preserves EV morphology and can be used in conjunction with phospholipid-conjugated stains with marginal interferences.

### EXAMPLE 6 - Increased stability of isolated EVs with NBI vs UC

Since biological materials are subjected to different storage conditions, we analysed the turnover of microvesicles stored at 4°C after purification by NBI or UC (Figure 6). In the 24-day post-isolation (t24) UC samples, the originally analysed 600 nm population was replaced by a ~ 300 nm population. Surprisingly, in the NBI samples 86% of the original EV population was still detectable using the same nanopore (Figure 6, in the center), and a systematic microvesicle analysis showed a half-life> 50 days for EVs from NBI, in contrast to 7.35 days for EVs from UC (Figure 6, right). In summary, NBI retains the original EV morphology and their dispersion resulting in better stability in solution.

### EXAMPLE 7 - Robustness of the NBI method in cellular systems

To evaluate the robustness of NBI in cellular systems, EVs were purified independently from U87 cell mediums seeded at different densities, in triplicate on 6-well plates (Fig 7). The recovered particles were proportional to the number of seeded cells, with a 6.14% (n = 9 for NBI 1, 2 and 3) global coefficient of variation (CV) for both the exosomes (197 ± 26 nm) and microvesicles ( 595 ± 37 nm); we did not observe a statistically significant variation (P = 0.459) between EVs from serum-free culture medium (NBI1) and vesicle-free, ultracentrifugate (dFBS) serum. Interestingly, the rapid NBI procedure applicable to small-volume allowed to follow vesicle release using fewer cells, such as 10³ cells/cm² (Figure 8). We observed a linear release of exosomes as a function of cell density, while a coherent release with different dynamics for microvesicles, possibly connected with different biogenetic mechanisms, stability and/or cell-mediated turnover related to particle size.

Isolated EVs were then compared with NBI method from MCF-7, PC3, MDA-MB-231 and SH-SY5Y tumor cell lines. In all cases, an equivalent distribution of vesicles of corresponding size was observed, except for SH-SY5Y cells that produced a weaker release of both vesicle populations (Fig. 9). NBI general versatility has also been demonstrated in the purification of Gram-negative bacteria (DH5α E. coli cells) produced vesicles, detecting a 92±29 nm diameter population (Figure 10).

### EXAMPLE 8 - NBI performance on liquid biopsies

Since EVs are released from many types of blood cells and could be studied as biomarkers, the performance of NBI on liquid biopsies from healthy donors with known counts of corpuscular elements was evaluated (Fig. 11). EV enrichment in plasma was observed compared to serum (Fig. 12) and NBI on plasma (0.5 ml) was systematically performed on 47 subjects with a 45±10 year average age. As shown in Fig. 13A, 30.56 ± 25.78*10⁹ exosomes/ml were recovered with a 249.5±36.71 nm average size and 5.49 ± 3.09*10⁸ microvesicles/ml with a 564.4 ± 57.3 nm average size, resulting in an exosomes/microvesicles ratio of about 55:1. The number of recovered exosomes correlates with the erythrocyte count (RBC) (Pearson r: 0.998 P value <0.0001), platelets (PLT, Pearson r: 0.958) and leukocytes (WBC, Pearson r: 0.970), while Pearson r drops to 0.74 or 0.72 with the% of the subspecies of eosinophils or basophils, respectively. On the other hand (Fig. 13B) the number of microvesicles is better correlated with the number of PLT (Pearson r: 0.989, P value <0.0001), RBC (Pearson r: 0.976) and WBC (Pearson r: 0.912), and has shown a low consistency with the percentage of eosinophils (Pearson r: 0.58) or basophils (Pearson r: 0.35).

Immunodepletion of purified vesicles using erythrocyte CD235a marker or platelet CD41a marker reduced the presence of exosomes or microvesicles in solution (Figure 14), indicating the possibility to estimate an EV physiological abundance in human plasma. Importantly, the positivity to specific membrane proteins could be used to order different "EV lines" based on the original cell types.

Finally, RNA extracted from 47 donor EVs and converted into cDNA was used to calculate the absolute number of GAPDH mRNA/~ 3*10⁹ EV for Droplet digital PCR assay (Fig. 15 and Fig. 16). The number of mRNA copies is correlated with PLT number (Pearson r: 0.623), possibly suggesting that a substantial amount of *GAPDH* mRNA was contained in microvesicles produced by PLT (Fig. 17), plasma cells being already recognized as leading producers of *GAPDH.* The analysis EV-contained/associated RNA was also carried out with droplet digital PCR, using the EvaGreen chemistry (BIO-RAD), without previous extraction of nucleic acids but using NBI-purified EVs directly encapsulated in oil-reaction droplets (Fig 18), demonstrating that the NBI method is compatible with downstream chemical reactions exploited by technologies for high sensitivity analysis of nucleic acids. The NBI method presented here is also compatible with other technologies used for the ultrasensitive detection of antigens (proteins) that may be present on EV surface. According to the principle of interactions between positive (metals such as nickel or aluminium) and negative (such as EV) net charges, NBI can be coupled to AlphaScreen (Perkin Elmer) technology, using nickel-chelated Acceptor beads and biotinylated antibodies recognized by Donor beads (Fig. 19A). EVs circulating in human plasma can thus be directly detected using antibodies shown in Fig. 19B, which represents the direct application of NBI associated with Alpha technology. The NBI method allows further verification of EV-associated protein presence by western blotting technique, of which experimental result is shown in Fig. 20 using antibodies that recognize proteins ubiquitously expressed in EVs.

In conclusion, NBI is the next-generation instrument for extracellular vesicle isolation.

### MATERIALS AND METHODS

### Cell cultures

U87-MG human glioma cells (ATCC^{®} HTB-14 ^{™}), SH-SY5Y dineuroblastoma cell lines (ATCC^{®} CRL-2266 ^{™}) and PC-3 prostatic adenocarcinoma (ATCC^{®} CRL-1435 ^{™}) were obtained from ATCC bank (American Type Culture Collection). The cell lines of mammary adenocarcinoma MCF7 (ICLC; HTL95021) and MDA-MB-231 (ICLC; HTL99004) were instead provided by the biological bank of the IRCCS Azienda Ospedaliera Universitaria San Martino - IST Istituto Nazionale per la Ricerca sul Cancro. These cells grow adherent, and except for PC-3 cells, which were kept in culture in RPMI 1640 medium, all the other lines were grown in DMEM medium, both added with 10% FBS (v/v), 100 U/ml penicillin + 100 ug/ml of streptomycin, 2 mM L-glutamine (Life Technologies, Carlsbad, CA, USA), and incubated at 37°C, with 5% CO₂. To obtain extracellular vesicle containing medium, the cells were initially cultured in whole medium until reaching 75% confluence (usually in 48 hours); subsequently, after having been gently washed twice with PBS, cells were incubated in a FBS-free medium for 24 hours. Cells were plated in different plate and flask formats according to the experiments to be carried out, but the density was kept constant at 3.2 ± 0.2 *10⁴/cm², unless otherwise described in the figure legend.

Before starting the NBI procedure, the collected culture medium was centrifuged at 2800 rcf for 10 minutes and gently transferred into new tubes. For the experiments described in Fig. 7, dFBS condition refers to the NBI carried out on a culture medium containing 100,000 rcf ultracentrifuged FBS, added to a 10%concentration. This culture medium was then 1:10 PBS diluted to reduce solution viscosity.

For cell density experiments in Fig. 9, U-87-MG, MDA-MB-231, SH-SY5Y, MCF7, and PC-3 cell lines were plated in triplicate in 6-well plates with the following well numbers: 3.4×10⁵; 1.7×10⁵; 8.5×10⁴; 4.2×10⁴; 2.1×10⁴ and 1.0×10⁴. After 48 hours incubation in whole medium, the cells were washed twice with PBS and incubated for 24 hours in FBS-free medium before continuing with the NBI protocol. In this case, after taking the EV-containing medium, the adhered cells were fixed with 4% paraformaldehyde, Hoechst 33342 labeled and washed with PBS before acquiring the images through a quantitative imaging system Operetta (Perkin Elmer). The images were acquired at 10X magnification and 50 fields per well were analysed by the Harmony software. The EVs were then analysed using Tunable Resistive Pulse Sensing using the qNano (IZON Science).

### EV isolation by differential ultracentrifugation

The EVs produced by U87-MG cells cultured in T150 flasks (CLS430823-50EA) were isolated by differential ultracentrifugation in accordance with the protocol described in Di (Vizio et al., Am J Pathol, 2012; 15: 1573-84) with minor modifications. Briefly, after 24 hours incubation in a FBS-free medium, the supernatants were collected in falcon tubes and centrifuged at 2,800 rcf at 4°C to remove cellular debris. The supernatants were then transferred into ultracentrifuge tubes (Polyallomer Quick-Seal centrifuge tubes 25 × 89 mm, Beckman Coulter) and centrifuged for 30 minutes at 4°C at 10000 rcf in an Optima XE-90 (Beckman Coulter) instrument with SW 32 Ti rotor. This step allowed preferential precipitation of microvesicles, which were gently re-suspended in filtered PBS. Then, according to the protocol described in Thèry C. et al. (Curr Protoc Cell Biol. 2006; Chapter 3: Unit 3.22), the collected supernatants were filtered through a 0.22 µm disposable filter (Sarstedt, Numbrecht, Germany) to remove microvesicle contaminants or aggregates, and centrifuged at 100,000 rcf for 70 min at 4°C to preferentially pellet exosomes. The pellets were re-suspended in filtered PBS. EVs obtained from differential ultracentrifugation were combined and stored at -80°C or kept at 4°C before being analysed by TRPS.

NBI reagents (preferably used):
PBS (ThermoFisher, 10010023) filtered with a 0.2 µm disposable filter membrane (used throughout the whole NBI protocol).
NiSO₄ [0.1 M] (Sigma, 656895)
NaCl [5 M] (Sigma, 450006)
Sodium citrate [0.2 M] (Sigma, C8532)
EDTA [0.5] M (ThermoFisher, UltraPure pH 8.0, 15575020)
KH₂PO₄ [1M] (Sigma, P9791)
Stripping buffer: PBS + 0.5 M NaCl, 50 mM EDTA pH 8.0
EV-Elution A to volume with PBS with a final concentration of 3.2 mM EDTA pH 8.0.
EV-Elution B: to volume with PBS with 60 mM NaCl, 45 µM sodium citrate.

After mixing EV-Elution buffer A and B (1X elution solution), 8 µl/ml KH₂PO₄ were added to the 1X solution before proceeding with EV elution.

### Microvesicle flow cytometry analysis

Vesicles from differential ultracentrifugation or from NBI were diluted in 0.22 µm filtered PBS. The background signal was set up based on the acquisition of the filtered PBS, and the light scattering threshold was corrected to allow an acquisition having an event rate of ≤5 events per second.

Light scattering detection was set in a logarithmic scale, the voltages assigned for the Forward Scattering and the Side Scattering were 300 and 310 V, respectively, and the threshold was set at 200 for both signals. The acquisition was performed at a low flow rate and the samples were carefully diluted to avoid swarm effect and coincidence of events. Standard 1 and 10 µm polystyrene beads (Invitrogen) were used to set the gates for microvesicles. When possible 10,000 events were counted for the analysis of each sample, on the basis of a time acquisition, at least 1 minute acquisition was recorded. Sample acquisition was performed with a FACS Canto flow cytometer (BD Biosciences) and data were analysed using the BD Diva (BD Biosciences) software.

### TRPS (Tunable Resistive Pulse Sensing)

EV size and concentration were characterized by TRPS using the qNano (IZON Science) tool. An average of 500 particles were counted for each sample, unless for 6-well plates experiments (Fig. 9, Fig. 3C and Fig. 8) or in the case of samples in which the particle rate was below 100 particles/min, in which at least 2 minutes of recording were analysed. NP200 nanopores (A40948, A43545, A43667, A43667), NP400 (A43592, A44117, A44116), NP800 (A40542, A36164, A40548, A44118) and N1000 (A40572) were used with a stretch between I 45.5 and I 47 mm. The voltage was set between 0.12-0.68 V to maintain a steady current intensity in the 95-130 nA range, with a background noise below 7-12 pA and a linear particle count rate. The calibration particles CPC100B (Batch ID: B8748N), CPC200B (Batch ID: B6481M), CPC500E (Batch ID: 659543B), CPC800E (Batch ID: 634561B) and CPC1000F (Batch ID: 669582B) with, respectively, 114 nm, 210 nm, 500 nm, 710 nm and 940 nm average diameter, were purchased by iZON Science. All data related to the analyses with qNANO were recorded and analysed by the Izon Control Suite v.3 software.

### Transmission Electron Microscopy (TEM)

Vesicles were visualized using a transmission electron microscope (TEM). Briefly, a 5 µl aliquot for each EV sample, fixed in elution buffer with 2.5% formaldehyde, was placed on a 300-square mesh grid in copper and nickel coated with a thin carbon film. Grids were then negatively labeled with a 1% uranyl acetate buffer at pH 4.5, and observed using a 100 kV TEM FEI Tecnai G2 Spirit microscope, equipped with an Olympus Morada camera (magnifications used: 20500x and 87000x).

Western blotting analyses were subsequently performed using anti-CD63 antibodies (Abcam, ab193349), anti-Flotillin-1 (BD Biosciences, 610821), and anti-Alix (Cell Signaling Technology, # 2171).

### Competitive assay

EV elution step was tested by a competitive assay in which 30 µg/ml of protein extract from DH5α E. coli and 15 µg/ml of recombinant proteins, tagged with histidine, purified (T7 RNA pol, 110 kDa; HuR, 36 kDa; YTH, 23 kDa) were added to10 ml medium containing EV derived from U87-MG cells. Briefly, DH5α cells were kept in culture in LB medium until an OD₆₀₀ of 0.5 was reached and were collected by centrifugation at 6000 rcf for 5 min. The pellet was re-suspended in 3 mL DMEM medium + 1 µg/ml lysozyme and sonicated at 4°C in a thermostatic bath for 7 cycles (40 ultrasound amplitude, 7 sec *on,* 10 sec off). The lysate was clarified by centrifugation at 13,000 rcf for 20 min and then filtered with a 0.2 µm disposable filter membrane before being added to the EV-containing medium. The recombinant protein tagged with histidine T7 RNA polymerase was kindly provided by Dr. S. Mansy's lab (CIBIO, University of Trento); the recombinant proteins HuR (D'Agostino et al., PLoS One, 2013 Aug 12; 8 (8): e72426) and YTH (Xu et al., J Biol Chem. 2015 Oct 9; 290: 24902-13) were produced and purified as described in the references.

NBI was performed following incubation times and reagents already described, except for elution gradient solutions indicated in Fig. 3B. Protein samples were quantified using the Bicinchoninic Acid Assay (BCA) and the Bradford assay, following the respective protocol instructions. Eluate equal volumes were loaded onto a 12% SDS-PAGE and subjected to a Sypro Ruby staining or western blotting using a 1:1000 dilution of a primary anti-histidine antibody (ab1187).

The number and size of the recovered particles were analysed by TRPS, respecting the sample heterogeneity, using NP800, NP400, and NP200 nanopores.

### EV isolation from Gram-negative bacteria

DH5α E. Coli cells were cultured in whole LB medium until OD₆₀₀ reached 0.7. The cells were pelleted at 4,000 rcf for 15 min and the supernatant was collected to be processed according to NBI protocol. The particles were counted through the qNANO instrument using the NP150 and NP200 nanopores.

### Liposome preparation

The liposome lipid composition, having a lipid composition similar to that of eukaryotic vesicles, is: 20% phosphatidylcholine moles, 10% phosphatidylethanolamine moles, 15% oleophosphatidylserine moles, 15% sphingomyelin moles, 40% cholesterol (Llorente et al. Biochim, Biophys, Acta 2013; 1831: 1302-9; Haraszti et al., J. Extracell, Vesicles 2016; 5: 32570) moles. Lipid films were created removing the organic solvent (e.g. chloroform) from the lipid solution by means of a rotary evaporator instrument and vacuum drying for at least 1 hour. Lipids, at 1 mg/mL final concentration, were re-suspended in DPBS and vigorously stirred with Vortex to form multilamellar liposomes, which were further exposed to 6 freeze-thaw cycles. The final liposome morphology was obtained by extruding a suspension of multilamellar liposomes using a two-syringe extruder (LiposoFast Basic Unit, Avestin Inc.). Thirty-one steps were performed through 2 stacked polycarbonate filters (Millipore) having pores of different size to obtain vesicles of different size (MacDonald et al. Biochim Biophys, Acta 1991; 1061: 297-303), subsequently verified by photon correlation spectroscopy with a Zeta Sizer instrument (Nano-ZS, Malvern Instruments).

### Blood samples

Whole blood samples from healthy donors were collected at Meyer Children's University Hospital. Plasma samples were collected in commercially available EDTA-treated tubes and then shipped from the hospital biological bank to the research laboratories according to cold chain. Informed consent was obtained by donors before sample analysis.

Plasma was obtained by removing cells after centrifugation for 10 minutes at 2,000 rcf using a refrigerated centrifuge (4°C) (Eppendorf 5702 R, Milan, Italy). Serum samples were obtained by allowing the blood to coagulate, leaving it undisturbed at room temperature for 30 minutes. Coagulum was removed by centrifuging at 2000 rcf using a refrigerated centrifuge (4°C) (Eppendorf 5702 R, Milan, Italy). The Complete blood count was analysed with a Sysmex XE-5000 flow cytometer (Sysmex America, Mundelein, IL). The analytical procedure was conducted according to the protocol instructions.

Immunodepletion of vesicles purified by NBI was performed using anti-CD235a (Miltenyl Biotec, 130-100-271) and anti-CD41a (Miltenyl Biotec, 130-105-608) biotinylated antibodies, respectively, and streptavidin beads (ThermoFisher, 11205D). The vesicles were analysed with qNano after the precipitation of the beads and normalized on the number of particles in the respective control samples with a quantitative equivalent of biotin (Sigma, B4501).

### RNA extraction

Total RNA was extracted using QlAzol reagent (QIAGEN), following the instructions enclosed with some modifications. Briefly, 100 µl of QlAzol were directly added to the beads before EV elution step, subsequently stirred with Vortex and incubated for 5 minutes at room temperature. Then 20 µl of chloroform were added. After vigorously mixing for 15 seconds, the samples were incubated at room temperature for 3 minutes. Phases were separated by centrifugation at 12,000 rcf for 15 minutes at 4°C, and the aqueous phase was drained out. After adding 1 µl glycogen (20 mg/ml) and 100 µl isopropanol, RNA was precipitated overnight at -80°C. After centrifugation at 12,000 rcf for 10 min, RNA pellets were washed with 75% ethanol, centrifuged as described above and re-suspended in 10 µl of RNase-free water. RNA was quantified using Bioanalyzer RNA 6000 Pico Kit (Agilent Technologies) following the protocol instructions.

### Reverse Transcription Reaction and Droplet Digital PCR

Reverse transcription reaction was performed using miRCURY LNA Universal RT microRNA PCR kit, Universal cDNA Synthesis Kit II (Exiqon) following the protocol instructions with the following reaction composition: 2.3 µl 5X reaction buffer, 1.15 µl enzyme mix, 0.5 µl synthetic RNA spike-in and 7.5 µl template total RNA. QX200TM Droplet DigitalTM PCR System (BioRad) was used to quantify GAPDH mRNA using EvaGreen chemistry and the following primers: 5'-CAACGAATTTGGCTACAGCA-3' (SEQ ID No. 1) and 5'-AGGGGTCTACATGGCAACTG-3' (SEQ ID No. 2).

### AlphaScreen assay

The reactions were performed in 384-Optiplate (Perkin Elmer) in a 20 µl final volume. Assay was optimized in PBS using 15 µg/ml nickel-chelate acceptor beads and 10 µg/ml streptavidin-donor beads with serial antibody dilutions to identify the attachment point. The presence of superficial markers was analysed in dose-response with EV serial dilution, previously characterized by TRPS. EVs were purified by NBI from healthy donor plasma or serum-free tumor cell samples. Fluorescence signal was finally detected by Enspire instrument (Perkin Elmer) after 90 minutes of incubation in the dark at room temperature.

### Statistical analysis

Data and number of independent experiments are indicated in the relevant captions of the figures. Anova, *t*-test, and the Pearson r coefficient were calculated by using GraphPad Prism v5.1 software, and the results were considered statistically significant when P value was <0.05 (*), <0.01 (**), < 0.001 (***).

## Claims

1. A stationary phase functionalized with cations selected from the group consisting of nickel and aluminium, and **characterized in that** it has a positive net charge between 30 and 80 mV; said stationary phase consisting of magnetic or non-magnetic particles of micrometric or nanometric size; wherein the stationary phase is selected from the group consisting of agarose or silicon beads, whether magnetic or non-magnetic, alginate salt matrices, polymers for Immobilized Metal ion Affinity Chromatography (IMAC), nickel chelate acceptor beads, anionic or carbon styrene polymers.

2. The stationary phase according to claim 1, with particles having an average size of 25-40 µm if in micrometer size.

3. A method for preparing the stationary phase according to any one of claims 1-2, said method comprising suspending a non-functionalized stationary phase in a saline solution buffered at physiological pH containing 15-100 mM of a nickel or aluminium salt.

4. The method according to claim 3 wherein the saline solution buffered at physiological pH is PBS or any other buffer with a pH between 7 and 7.5.

5. The method according to anyone of claims 3-4 wherein the suspension is incubated at room temperature with gentle orbital rotation.

6. A method for isolating extracellular vesicles (EVs), secreted by eukaryotic or prokaryotic cells in a biological liquid, said method comprising the use of the stationary phase functionalized with cations selected from the group consisting of nickel and aluminium, and **characterized in that** it has a positive net charge between 30 and 80 mV; said stationary phase consisting of magnetic or non-magnetic particles of micrometric or nanometric size.

7. The method according to claim 6 wherein the stationary phase is according to any one of claims 1-2.

8. The method according to claim 6 or 7, wherein the stationary phase is added dropwise to the surface of a biological liquid.

9. The method according to any one of claims 6-8 wherein, after incubation in a biological liquid, the beads are separated by gentle centrifugation and decantation.

10. The method according to any one of claims 6-9 wherein EVs are removed from the stationary phase by incubation in an at least equal volume of an Elution solution prepared a few minutes before use by mixing two saline solutions at physiological pH containing at least two different chelating agents.

11. The method according to claim 10 wherein the chelating agents are selected from the group consisting of EDTA and sodium citrate.

12. The method according to claim 10 wherein, in the final Elution solution, EDTA has a 3-6 mM concentration and sodium citrate has a 1-300 µM concentration.

13. The method according to any one of claims 9-11 wherein the incubation with Elution solution is maintained under orbital rotation at 20-37°C.

14. The method according to any one of claims 6-13 wherein the biological liquid is selected from the group consisting of cell culture media, physiological buffer solutions, bacterial culture media, human-animal blood, human-animal tissue exudates.

15. The method according to any one of claims 6-14 in which the isolated EVs are then subjected to protocols for protein and nucleic acid extraction (DNA, RNA) from EVs, antigen detection technologies in combination with specific antibodies, detection and quantification of associated and/or vesicle-contained nucleic acids by means of polymerase chain reaction (RT-PCR), detection and quantification of associated and/or vesicle-contained nucleic acids by droplet digital PCR.

16. A kit comprising:
a container containing a stationary phase according to any one of claims 1-2 or,
a container containing a non-functionalized stationary phase consisting of magnetic and non-magnetic particles of micrometric or nanometric size; wherein the stationary phase is selected from the group consisting of agarose or silicon beads, whether magnetic or non-magnetic, alginate salt matrices, polymers for Immobilized Metal ion Affinity Chromatography (IMAC), nickel chelate acceptor beads, anionic or carbon styrene polymers;
a container containing a nickel or aluminium salt, and a container comprising physiological phosphate buffer saline (PBS) solution.

17. The kit according to claim 16, further comprising:
at least two containers each containing a different chelating agent.

## Patentansprüche

1. Stationäre Phase, die mit Kationen funktionalisiert ist, ausgewählt aus der Gruppe bestehend aus Nickel und Aluminium, und **dadurch gekennzeichnet ist, dass** sie eine positive Nettoladung zwischen 30 und 80 mV aufweist; wobei die stationäre Phase aus magnetischen oder nichtmagnetischen Teilchen von mikrometrischer oder nanometrischer Größe besteht; wobei die stationäre Phase ausgewählt ist aus der Gruppe bestehend aus magnetischen oder nichtmagnetischen Agarose- oder Silikonperlen, Alginatsalzmatrizen, Polymeren für die immobilisierte Metallionen-Affinitätschromatographie (IMAC), Nickelchelat-Akzeptorperlen, anionischen oder Kohlenstoff-Styrol-Polymeren.

2. Stationäre Phase nach Anspruch 1, wobei die Partikel eine durchschnittliche Größe von 25 - 40 µm aufweisen, wenn sie im Mikrometerbereich liegen.

3. Verfahren zur Herstellung der stationären Phase nach einem der Ansprüche 1 bis 2, wobei das Verfahren das Suspendieren einer nicht funktionalisierten stationären Phase in einer bei physiologischem pH-Wert gepufferten Salzlösung umfasst, die 15 bis 100 mM eines Nickel- oder Aluminiumsalzes enthält.

4. Verfahren nach Anspruch 3, wobei die bei physiologischem pH-Wert gepufferte Salzlösung PBS oder ein anderer Puffer mit einem pH-Wert zwischen 7 und 7,5 ist.

5. Verfahren nach einem der Ansprüche 3 bis 4, wobei die Suspension bei Raumtemperatur unter leichter orbitaler Rotation inkubiert wird.

6. Verfahren zur Isolierung von extrazellulären Vesikeln (EVs), die von eukaryotischen oder prokaryotischen Zellen in einer biologischen Flüssigkeit sezerniert werden, wobei das Verfahren die Verwendung einer stationären Phase umfasst, die mit Kationen funktionalisiert ist, ausgewählt aus der Gruppe bestehend aus Nickel und Aluminium, und **dadurch gekennzeichnet ist, dass** sie eine positive Nettoladung zwischen 30 und 80 mV aufweist; wobei die stationäre Phase aus magnetischen oder nichtmagnetischen Teilchen von mikrometrischer oder nanometrischer Größe besteht.

7. Verfahren nach Anspruch 6, wobei die stationäre Phase einem der Ansprüche 1 bis 2 entspricht.

8. Verfahren nach Anspruch 6 oder 7, wobei die stationäre Phase tropfenweise auf die Oberfläche einer biologischen Flüssigkeit gegeben wird.

9. Verfahren nach einem der Ansprüche 6 bis 8, wobei nach der Inkubation in einer biologischen Flüssigkeit die Perlen durch sanftes Zentrifugieren und Dekantieren getrennt werden.

10. Verfahren nach einem der Ansprüche 6 bis 9, wobei EVs aus der stationären Phase durch Inkubation in einem mindestens gleichen Volumen einer Elutionslösung entfernt werden, die einige Minuten vor der Verwendung durch Mischen von zwei Kochsalzlösungen mit physiologischem pH-Wert, die mindestens zwei verschiedene Chelatbildner enthalten, hergestellt wird.

11. Verfahren nach Anspruch 10, wobei die Chelatbildner ausgewählt sind aus der Gruppe bestehend aus EDTA und Natriumcitrat.

12. Verfahren nach Anspruch 10, wobei in der endgültigen Elutionslösung EDTA eine Konzentration von 3 - 6 mM und Natriumcitrat eine Konzentration von 1 - 300 µM aufweist.

13. Verfahren nach einem der Ansprüche 9 bis 11, wobei die Inkubation mit der Elutionslösung unter orbitaler Rotation bei 20 - 37 °C gehalten wird.

14. Verfahren nach einem der Ansprüche 6 bis 13, wobei die biologische Flüssigkeit ausgewählt ist aus der Gruppe bestehend aus Zellkulturmedien, physiologischen Pufferlösungen, bakteriellen Kulturmedien, menschlich-tierischem Blut und menschlich-tierischen Gewebeexsudaten.

15. Verfahren nach einem der Ansprüche 6 bis 14, wobei die isolierten EVs dann Protokollen für die Extraktion von Proteinen und Nukleinsäuren (DNA, RNA) aus EVs, Antigen-Nachweistechnologien in Kombination mit spezifischen Antikörpern, Nachweis und Quantifizierung von assoziierten und/oder Vesikel-enthaltenden Nukleinsäuren mittels Polymerase-Kettenreaktion (RT-PCR), Nachweis und Quantifizierung von assoziierten und/oder Vesikel-enthaltenden Nukleinsäuren durch digitale Tröpfchen-PCR unterzogen werden.

16. Kit umfassend:
einen Behälter, der eine stationäre Phase nach einem der Ansprüche 1 bis 2 enthält, oder
einen Behälter, der eine nicht-funktionalisierte stationäre Phase enthält, die aus magnetischen und nicht-magnetischen Teilchen mikrometrischer oder nanometrischer Größe besteht; wobei die stationäre Phase ausgewählt ist aus der Gruppe bestehend aus magnetischen oder nicht-magnetischen Agarose- oder Silikonperlen, Alginatsalzmatrizen, Polymeren für immobilisierte Metallionen-Affinitätschromatographie (IMAC), Nickelchelat-Akzeptorperlen, anionischen oder Kohlenstoff-Styrol-Polymeren;
einen Behälter, der ein Nickel- oder Aluminiumsalz enthält, und
einen Behälter mit physiologischer Phosphatpuffersalz(PBS)-Lösung.

17. Kit nach Anspruch 16, ferner umfassend:
mindestens zwei Behälter, die jeweils einen anderen Chelatbildner enthalten.

## Revendications

1. Phase stationnaire fonctionnalisée avec des cations choisis dans le groupe constitué par le nickel et l'aluminium, et **caractérisée en ce qu'**elle a une charge nette positive comprise entre 30 et 80 mV ; ladite phase stationnaire étant constituée de particules magnétiques ou non magnétiques de taille micrométrique ou nanométrique ; dans laquelle la phase stationnaire est choisie dans le groupe constitué de billes d'agarose ou de silicium, magnétiques ou non magnétiques, de matrices de sel d'alginate, de polymères pour la chromatographie d'affinité par ions métalliques immobilisés (IMAC), de billes acceptrices de chélates de nickel, de polymères de styrène anioniques ou de carbone.

2. Phase stationnaire selon la revendication 1, avec des particules ayant une taille moyenne de 25-40 µm si en taille micrométrique.

3. Procédé de préparation de la phase stationnaire selon l'une quelconque des revendications 1 et 2, ledit procédé comprenant la mise en suspension d'une phase stationnaire non fonctionnalisée dans une solution saline tamponnée à un pH physiologique contenant 15-100 mM d'un sel de nickel ou d'aluminium.

4. Procédé selon la revendication 3, dans lequel la solution saline tamponnée au pH physiologique est du PBS ou tout autre tampon dont le pH est compris entre 7 et 7,5.

5. Procédé selon l'une quelconque des revendications 3 et 4 dans lequel la suspension est incubée à température ambiante avec une légère rotation orbitale.

6. Procédé d'isolement des vésicules extracellulaires (VE) sécrétées par des cellules eucaryotes ou procaryotes dans un liquide biologique, ledit procédé comprenant l'utilisation de la phase stationnaire fonctionnalisée avec des cations choisis dans le groupe constitué par le nickel et l'aluminium, et **caractérisé en ce qu'**il a une charge nette positive entre 30 et 80 mV ; ladite phase stationnaire étant constituée de particules magnétiques ou non magnétiques d'une taille micrométrique ou nanométrique.

7. Procédé selon la revendication 6, dans lequel la phase stationnaire est conforme à l'une quelconque des revendications 1 et 2.

8. Procédé selon la revendication 6 ou 7, dans lequel la phase stationnaire est ajoutée goutte à goutte à la surface d'un liquide biologique.

9. Procédé selon l'une quelconque des revendications 6 à 8 dans lequel, après incubation dans un liquide biologique, les billes sont séparées par centrifugation douce et décantation.

10. Procédé selon l'une quelconque des revendications 6 à 9, dans lequel les VE sont éliminées de la phase stationnaire par incubation dans un volume au moins égal d'une solution d'élution préparée quelques minutes avant l'utilisation en mélangeant deux solutions salines à pH physiologique contenant au moins deux agents chélatants différents.

11. Procédé selon la revendication 10, dans lequel les agents chélateurs sont choisis dans le groupe constitué par l'EDTA et le citrate de sodium.

12. Procédé selon la revendication 10 dans lequel, dans la solution d'élution finale, l'EDTA a une concentration de 3-6 mM et le citrate de sodium a une concentration de 1-300 µM.

13. Procédé selon l'une quelconque des revendications 9 à 11, dans lequel l'incubation avec la solution d'élution est maintenue sous rotation orbitale à 20-37 °C.

14. Procédé selon l'une quelconque des revendications 6 à 13, dans lequel le liquide biologique est choisi dans le groupe constitué par les milieux de culture cellulaire, les solutions tampons physiologiques, les milieux de culture bactérienne, le sang humain-animal, les exsudats de tissus humains-animaux.

15. Procédé selon l'une quelconque des revendications 6 à 14 dans lequel les VE isolées sont ensuite soumises à des protocoles d'extraction de protéines et d'acides nucléiques (ADN, ARN) des VE, à des technologies de détection d'antigènes en combinaison avec des anticorps spécifiques, à la détection et à la quantification des acides nucléiques associés et/ou contenus dans les vésicules au moyen d'une réaction en chaîne par polymérase (RT-PCR), à la détection et à la quantification des acides nucléiques associés et/ou contenus dans les vésicules au moyen d'une PCR numérique en gouttelettes.

16. Kit comprenant :
un récipient contenant une phase stationnaire selon l'une quelconque des revendications 1 et 2, ou
un récipient contenant une phase stationnaire non fonctionnalisée constituée de particules magnétiques et non magnétiques de taille micrométrique ou nanométrique ; la phase stationnaire étant choisie dans le groupe constitué de billes d'agarose ou de silicium, magnétiques ou non magnétiques, de matrices de sel d'alginate, de polymères pour la chromatographie d'affinité sur ions métalliques immobilisés (IMAC), de billes acceptrices de chélates de nickel, de polymères de styrène anioniques ou de carbone ;
un récipient contenant un sel de nickel ou d'aluminium, et
un récipient contenant une solution physiologique de tampon phosphate salin (PBS).

17. Kit selon la revendication 16, comprenant en outre :
au moins deux récipients contenant chacun un agent chélatant différent.
